# EUROPEAN PATENT SPECIFICATION

(11) **EP 0 812 181 B1**
(45) Date of publication and mention of the grant of the patent: **04.10.2000**
(21) Application number: 95908839.4
(22) Date of filing: 28.02.1995
(51) Int. Cl.: A61K 7/22

(54) **COMPOSITION FOR HYGIENE, PROPHYLAXIS AND TREATMENT OF THE ORAL CAVITY**
ZUSAMMENSETZUNG ZUR HYGIENE,PROPHYLAXE UND BEHANDLUNG DER MUNDHÖHLE
COMPOSITION D'UNE PREPARATION HYGIENIQUE, PROPHYLACTIQUE ET THERAPEUTIQUE POUR LA CAVITE BUCCALE

(43) Date of publication of application: 17.12.1997
(73) Proprietor: U.S. Meds. - Bulgaria Ltd., 1408 Sofia (BG); Grenville Financial Company Ltd., 1066 Nicosia (CY)
(72) Inventor: NIKOLAEV, Nikolay, Nikolaev, 1408 Sofia (BG)
(74) Representative: Tiedtke, Harro, Dipl.-Ing.
(86) International application number: BG9500001
(87) International publication number: WO9626707

(56) References cited:
- US-A- 5 104 644

## Description

The present invention refers to the composition for hygiene of the oral cavity, prophylaxis of the diseases connected with the tooth enamel and the formation of caries, disinfection and treatment of the inflammatory processes, restoring the tissue in the oral cavity and preventing plaque and tartar formation.

### Prior Art

Various preparations for hygiene and prophylaxis of the oral cavity are known. Their active ingredients keep effective control over the etiological factors which cause the diseases in the oral cavity. They also control the micro flora of the oral cavity, inflammations, plaque and other local factors which help the individual to improve his current condition when he has dental and oral diseases.

The effect of the hydrogen peroxide as an active ingredient for hygiene, prophylaxis and treatment of the oral cavity diseases is well known. Under the influence of the enzymes catalase and peroxidase the hydrogen peroxide breaks down and liberates "active" oxigen which suppresses the progress of the anaerobic bacteria and helps the progress of the "healthy" micro flora in the oral cavity. Thus it disinfects and restores the tissue in the oral cavity to its normal state (Wennström J. and Lindhe J. "Effects of Hydrogen Peroxide on Developing Plaque and Gingivitis in Man" - Journal of Clinical Periodontology, 1979, No.6, pages 115-130).

The effect of the zinc chloride as an antibacterial agent is well known. It combines with the bacterial cell membranes, suppresses the bacterial metabolism and inhibits the growth. It also reduces the adhesion between the bacteria and the teeth, protects the tooth enamel from food and from the acids which are products of the bacterial metabolism (Gedalia I. and com. "Effect of Zinc Chloride Mouthwash on Plaque and Zinc Levels of Surface Enamel" - Clinical Preventive Dentistry Vol.10, No.4, 1988, pages 3-5).

It is known that the combination of these active components in a composition used for rinsing and hygiene of the oral cavity and in a certain concentration is appropriate for treatment of periodontosis and stomatitis (US-A-5104644 dated April 14, 1992).

The composition consists of the following active ingredients in weight per cent:
Hydrogen peroxide - from 0,5 to 3,0
Zinc chloride - over 0,02, preferable from 0,02 to 0,8
Sodium citrate - over 0,03, preferable from 0,03 to 0,2
Sodium lauril sulfate - over 0,04, preferable from 0,04 to 0,025
Ethanol - from 2,0 to 3,5

The remaining is deionized water and ingredients which add fragrance and flavor to the water.

The pH of the preparation is about 3,5-4,0, in some cases up to 5,5.

The combination of the active ingredients has an antibacterial effect, reduces the plaque, regulates the cells' metabolism, inhibits the inflammation agents and helps the restore of the tissue, stops bleeding and also prevents periodontosis, stomatitis, gingivitis and inflammation.

According to the purpose of the composition, three types of concentration of the active ingredients are recommended: the smallest concentration - for rinsing and prophylaxis of the oral cavity; the average concentration - for reducing the plaque; the biggest concentration - for treatment of periodontosis.

### Brief description of the Invention

The problem to be solved with the preparations for prophylaxis and treatment of the oral cavity is to insure their harmlessness and maintain a physiological bacterial balance in the oral cavity. The combination of active ingredients may cause not only destruction of the disease connected bacteria, but also of the bacteria which are part of the normal flora of the oral cavity. This also cause a change of the taste perception in the oral cavity. Another problem is the staining of the teeth and the secondary development of some microorganisms like candida albicans.

This problem is solved by the present invention which is a preparation for hygiene, prophylaxis and treatment of the oral cavity. The preparation is composed of main active ingredients: hydrogen peroxide and zinc chloride and added to them tyloxapol and tartaric acid in the following proportion in weight per cent:

| | |
|---|---|
| Hydrogen peroxide | from 0.5 to 2.0 |
| Zinc chloride | from 0.02 to 0.65 |
| Tyloxapol | from 0.03 to 0.07 |
| Tartaric acid | from 0.01 to 0.03 |
| Ethanol | from 2.5 to 5.0 |

The remainder to 100% is deionized water and some ingredients for fragrance, flavor and stabilization.

In present invention it was discovered that the tyloxapol, which is a polymer of Oxyethylated tertiary octylphenol formaldehyde i.e. [4-(1,1,3,3-tetramethylbutyl)-phenol polymer with formaldehyde and oxirane) - Merck Index 10, 9632, and which is well known as aerosol detergent for the treatment of pulmonary diseases even of the newborn (Miller J.B. "Detergent Aerosol Therapy" - Clinical Medicine, Oct. 1967, No.74, pages 37-40), combined with hydrogen peroxide, zinc chloride and tartaric acid can be used for prophylaxis and treatment of the oral cavity diseases. Its selective effect on the pathogenic bacteria including candida albicans and consequently maintaining the physiological balance of the microorganisms in the oral cavity was discovered for the first time. The combination with tartaric acid intensifies this effect.

The advantage of the invention is the presence of the ingredient tyloxapol which is a non-ionic detergent and absolutely harmless. Used in combination with zinc chloride it reduces the surface tension and prevents the formation of cells' bacterial clusters, which cause stomatitis, gingivitis and periodontosis. In addition it inhibits the bacterial enzymes and increases the permeability of the bacterial membranes. The combination has marked synergetic suppressive effect on the growth of the pathogenic bacteria.

In the preparation the tartaric acid acts as a synergist in suppressing the inflammatory processes in the oral cavity. It also increases the membrane permeability and has antibacterial effect. In this case is possible to add and some citric acid in quantity from 1 to 7 times to tartaric acid.

The preparation as a whole, depending on its concentration has a healing effect, chiefly because it suppresses the inflammatory processes. It also reduces the bleeding of the gums, helps to maintain a bacterial balance in the oral cavity especially with patents who have had traumas after dental treatment, eases the cleaning of the teeth and dentures and protects from inflammation after the insertion of dentures, reduces the tartar and refreshes the oral cavity. When used for a long period of time it is absolutely harmless. This quality is chiefly due to the tyloxapol.

### Detailed descrirtion of the Invention

The following examples give detailed information for the invention without restricting it:

### Example 1

The underwritten ingredients are mixed in a quantity weight per cent, as their eventual change is possible in the limits shown in the second column:

| | |
|---|---|
| Ethyl alcohol | 3,00 ± 1,000 , |
| Glycerine | 3,00 ± 0,500 , |
| Poloxamer 407 | 0,30 ± 0,100 , |
| Saccharine | 0,03 ± 0,050 , |
| Tyloxapol | 0,05 ± 0,020 , |
| Citric acid | 0,05 ± 0,020 , |
| Tartaric acid | 0,02 ± 0,010 , |
| Zinc chloride | 0,03 ± 0,010 , |
| Aromatic composition (Menthol) | 0,03 ± 0,005 , |
| Na - EDTA | 0,04 ± 0,010 , |
| Hydrogen peroxide | 0,60 ± 0,100 , |

Deionized water to 100 and drops of sodium hydroxide for regulating the pH of the solution from 3,5 to 4,0.

The solution in example 1 must be put in packages, stored at a room temperature (from 5°C to 35°C) and used for prophylaxis of the oral cavity. After brushing of the teeth the mouth cavity is rinsed 3 times per day with 15ml. mouthwash which is measured by the dosage cup.

### Example 2

Solution identical to the one quoted in example 1 should be prepared but the quantity of the zinc chloride and hydrogen peroxide should be increased as follows:
- Zinc chloride: 0,6 w.p.c. ±0,05 ,
- Hydrogen peroxide: 1,5 w.p.c. ±0,1

The preparation is appropriate for prophylaxis and treatment of periodontosis, bleeding gums and traumas in the oral cavity. The solution in example 2 is used in the same way as the one in example 1 but no longer than 20 days. After finishing the active treatment one should go on with the solution in example 1 which is used for prophylaxis of the oral cavity.

### Example 3

The production of the preparation is made by the consecutive mixing of the ingredients. For production of 1000 kg. of the preparation the ingredients should be mixed and stirred incessantly in a stainless container in the following order:

| | |
|---|---|
| Ethyl alcohol | 15,00 kg, |
| Glicerine | 30,00 kg, |
| Zinc chloride | 0,18 kg, |
| Na - EDTA | 0,44 kg, |
| Tartaric acid | 0,24 kg, |
| Citric acid | 1,60 kg, |
| Saccharine | 0,18 kg, |
| Poloxamer 407 | 4,00 kg, |
| Ethyl alcohol | 15,00 kg, |
| Tyloxapol | 0,75 kg, |
| Aromatic composition | 0,36 kg, |
| Deionized water | 832,25 kg, |
| Hydrogen peroxide 6% | 100,00 kg. |

### Example 4

In this example the irritating effect of the preparation on the mucous membrane of the golden hamsters is examined. Thirty animals were involved in the research, fifteen were treated with tap water and fifteen with the preparation (brand name "Sure Choice").

In the begining of the research each animal was anesthetized with 0,2 cc Ketamin HCl intraperitoneal. The mucous membrane of the left cheek was scraped off by metal brush. The cotton swabs were soaked in the preparation and in the tap water and were applied for 10 minutes on the left cheek (the experimental one) and the right cheek (the control one). They were treated for 13-14 hours, 30 consecutive days. The scraps of the mucous membrane of the left and the right cheek were fixed in 10% neutral formalin and were included in paraffin blocks. Staining with histological preparation was made on 5 microns thick histological cuts. They were examined for: corrosion, inflammation changes, epithelial hyperplasia and fibrosis.

Photographs of the left cheek of the animals were taken with Minolta camera and Kodak Gold film. The results were processed by a computer program based on the method of the variation analisis with probability range p<0,5. The results show the following:

Judging by the histological change in the mechanically injured mucous membrane of the hamsters, the tested preparation shows good anti-inflammation effect.

Compared to the control side the preparation shows fibrosis effect in the submocosa.

Under the influence of the preparation the mucous membrane 30 days after the mechanical injury regenerates completely without any hyperplastic activities of the cells' layers and the keratin.

### Example 5

In this experiment the skin sensitivity potential of the preparation was examined.

The research was performed on white guinea-pigs according to the requirements of FAO for testing skin sensitivity (Report BSR 7/LO2). Examined were the following group of animals: Preliminary screening - 5 animals; negative controls - 4 animals; positive controls - tread with 0,1% 2,4 dinitrochloridebenzole in 80% water solution of ethanol - 6 animals; test animals, treated with the preparation - 10 animals.

The preliminary screening was made five times per week 8 hours per day. At the end of the exposure and at the end of the 24th hour skin erythema and skin edema were observed.

Research examination for induction was also made and the preparation was applied the first day for 24 hours and after that it was applied for 8 hours during three weeks, three days each week. Skin erythema and skin edema were observed.

The same experiment was made with positive controls. The preparation was applied in two weeks. The negative control group received permitted doze from the preparation without preliminary induction. The duration of the exposure was 24 hours. The animals were examined in a week after applying the permitted dozes. The reading of the data was on the 24th and the 48th hour from the applying of the due permitted doses.

The examination of the skin sensitivity of the concentrated preparation for prophylaxis of the oral cavity shows that after the dual application of the permitted dozes there are no positive skin reactions.

The preparation does not show any contact activity when tested according to the procedure of FAO on white guinea-pigs.

## Claims

1. Composition of the preparation for hygiene, prophylaxis and treatment of the oral cavity based on hydrogen peroxide, zinc bichloride and ethanol, supplemented by the existence of tiloxapol and also tartaric acid in the following quantity proportion in per cent by weight:
| | |
|---|---|
| Hydrogen peroxide | from 0,5 to 2,0 |
| Zinc bichloride | from 0,02 to 0,65 , |
| Tiloxapol | from 0,03 to 0,07 , |
| Tartaric acid | from 0,01 to 0,03 , |
| Ethanol | from 2,5 to 5,0 |
And the remainder to 100% is deionized water and ingredients for fragrance, flavor and stabilization.

2. Composition according to claim 1, characterized by the preference for the hydrogen peroxide to be from 0,5 to 0,7 weight per cent and the zinc bichloride from 0,02 to 0,04 weight per cent.

3. Composition according to claim 1, characterized by the preference for the hydrogen peroxide to be from 1,4 to 1,6 weight per cent and the zinc bichloride from 0,55 to 0,65 weight per cent.

4. Composition of the preparation for hygiene, prophylaxis and treatment of the oral cavity like it is shown in the description and the examples for realisation.

## Patentansprüche

1. Zusammensetzung des Präparates für die Hygiene, die Prophylaxe und die Behandlung der Mundhöhle, das auf Wasserstoffperoxid, Zinkdichlorid und Ethanol basiert, wobei es ergänzt ist durch das Vorhandensein von Tyloxapol und ebenso Weinsäure in den folgenden Mengenverhältnissen in Gewichtsprozent:
| | |
|---|---|
| Wasserstoffperoxid | von 0,5 bis 2,0, |
| Zinkdichlorid | von 0,02 bis 0,65, |
| Tyloxapol | von 0,03 bis 0,07, |
| Weinsäure | von 0,01 bis 0,03, |
| Ethanol | von 2,5 bis 5,0; |
und der Rest auf 100% deionisiertes Wasser und Inhaltsstoffe für den Duft, den Geschmack und die Stabilisierung ist.

2. Zusammensetzung nach Anspruch 1, gekennzeichnet durch die Bevorzugung, dass das Wasserstoffperoxid von 0,5 bis 0,7 Gewichtsprozent und das Zinkdichlorid von 0,02 bis 0,04 Gewichtsprozent ist.

3. Zusammensetzung nach Anspruch 1, gekennzeichnet durch die Bevorzugung, dass das Wasserstoffperoxid von 1,4 bis 1,6 Gewichtsprozent und das Zinkdichlorid von 0,55 bis 0,65 Gewichtsprozent ist.

4. Zusammensetzung des Präparates für die Hygiene, die Prophylaxe und die Behandlung der Mundhöhle, wie es in der Beschreibung und den Beispielen zur Realisierung gezeigt ist.

## Revendications

1. Composition d'une préparation pour l'hygiène, la prophylaxie et le traitement de la cavité buccale, à base de peroxyde d'hydrogène, de bichlorure de zinc et d'éthanol, complétée par la présence de tyloxapol et aussi d'acide tartrique dans les proportions quantitatives suivantes, en pourcentage en poids :
| | |
|---|---|
| Peroxyde d'hydrogène | de 0,5 à 2,0, |
| Bichlorure de zinc | de 0,02 à 0,65, |
| Tyloxapol | de 0,03 à 0,07, |
| Acide tartrique | de 0,01 à 0,03, |
| Ethanol | de 2,5 à 5,0 |
le complément jusqu'à 100 % étant constitué d'eau désionisée et d'ingrédients de parfum, d'arôme et de stabilisation.

2. Composition selon la revendication 1, caractérisée par des teneurs préférées en peroxyde d'hydrogène de 0,5 à 0,7 % en poids et en bichlorure de zinc de 0,02 à 0,04 % en poids.

3. Composition selon la revendication 1, caractérisée par des teneurs préférées en peroxyde d'hydrogène de 1,4 à 1,6 % en poids et en bichlorure de zinc de 0,55 à 0,65 % en poids.

4. Composition d'une préparation pour l'hygiène, la prophylaxie et le traitement de la cavité buccale, telle que définie dans la description et les exemples de réalisation.
